# EUROPEAN PATENT APPLICATION

(11) **EP 1 918 257 A1**
(43) Date of publication of application: **07.05.2008**
(21) Application number: 07075860.2
(22) Date of filing: 04.10.2007
(51) Int. Cl.: C02F 11/02, A01K 67/033

(54) **Method for releasing aquatic worms form a carrier and predation reactor**

(30) Priority: 04.10.2006 NL 1032621
(71) Applicant: Stichting Wetsus Centre of Excellence for Sustainable Water Technology, 8934 CJ Leeuwarden (NL)
(72) Inventor: Elissen, Hellen Johannes Hubertina, 6668 AT Randwijk (NL); Temmink, Bernard Gerhard, 6701 CP Wageningen (NL)
(74) Representative: Bruin, Cornelis Willem

(57) **Abstract**

The invention relates to a method comprising providing a carrier, preferably a porous carrier, comprising a number of aquatic worms from the class of the *Oligochaeta* exhibiting a swimming motion. The method further comprises providing to the worms an escape reflex-inducing stimulus on at least one side of the carrier of, such that the worm biomass is at least partially released from the carrier. The method further comprises removing worm biomass released from the porous carrier for further processing. The invention further relates to a predation reactor suitable for applying the method according to the invention.

## Description

The present invention according to a first aspect relates to a method for removing aquatic worms from a carrier.

According to a further aspect, the invention relates to a predation reactor, wherein aquatic worms from the class of the Oligochaeta are used.

Worms from the class of the *Oligochaeta* are extremely suitable for application in reducing and compacting sludge, which is produced in both communal and industrial water purification plants. With the sludge as a food source, the worms may grow and replicate. Some species do this by means of (asexual) division. They may thus form a valuable protein rich raw material for further processing to for example fish feeds or coatings. Separation of sludge and worms provides a great added value to such purification systems, over systems wherein the worm biomass is recirculated.

For the processing of sludge, the aquatic worms of the class of the *Oligochaeta* for example are used in a reactor wherein a porous carrier, such as a net or cloth having a fine mash or a porous three dimensional carrier. In the openings (pores) of the porous carrier, the worms may nest and from there may feed on constituents of the sludge, which flows along and/or through the pores. Hereby the worm biomass in the porous carrier increases.

A bottleneck in the application of the worm biomass however, is the removal of the worms from the carrier material. Thus far, aquatic worms, applied in a reactor from sludge reduction, can insufficiently be removed from such a carrier material. Finding a solution for the problem of the separation of carrier material and worm biomass thus is of great importance.

The inventors of the present invention have now found that aquatic worms from the class of the *Oligochaeta,* which are capable of motion by swimming, may be removed from carrier materials, for example porous carrier materials, by inducing a so called escape reflex. An escape reflex is a neurophysiological reaction which occurs in certain *Oligochaeta* in response to exposure to sublethal concentrations of toxins or touching. C.D. Drewes, (Sublethal effects of environmental toxicants on oligochaete escape reflexes, Amer. Zool., 37:346-353 (1997)) describes the background of the escape reflex in the *Oligochaeta Lumbriculus terrestris, Eisenia foetida,* and *Lumbriculus variegatus.*

The inventors at the present invention have now found that such an escape reflex, for example in reaction to a chemical stimulus, may also occur in other aquatic worms from the order of the *Oligochaeta*, which exhibit a swimming motion. Meaning in those Oligochaete aquatic worms, which are capable of motion by means of a helix-like movement, analogous to what is described by Drewes and Fourtner (Helical swimming in a fresh water Oligochaete., Biol. Bull. 185:1-9 (1993)) or by Drewes (Helical swimming and body reversal behaviours in Lumbriculus variegatus., Hydrobiologia 406: 263-269 (1999)).

Furthermore, the inventors of the present invention have now found that the escape reflex of the *Oligochaeta* may be used to release such aquatic worms from the order of the *Oligochaeta* which exhibit a swimming motion from a carrier, such as a porous carrier. The invention thus relates to a method according to claim 1.

In the method according to claim 1 a carrier comprising aquatic worms from the class of the *Oligochaeta* exhibiting swimming motion is provided. As the porous carrier any material may be suitable, wherein or whereon the aquatic worms exhibiting a swimming motion may establish and from where they may be removed by a swimming motion. Examples of suitable carriers are carriers provided with openings (inclusive pours) or prefaces, wherein or whereon the worms may establish, flexible spongelike materials, such as Recticel^{®} or mashlike materials. According to a preferred embodiment a porous carrier is applied, more preferably having a mean pore size of 100 µm till several mm, such as 100-3000 µm, for example 200-1000 µm, more preferably 200-500 µm.

The aquatic worms exhibiting a swimming motion may be selected form the order of the *Oligochaeta,* preferably from the family of the Lumbriculidae or the family of the Naididae, such as *Nais variabilis* or *Nais simplex.* More preferably the worms are selected from the genus *Lumbriculus*, such as the species *Lumbriculus variegatus* or from the genus *Dero*, for example the species *Dero digitata. Lumbriculus variegatus* has shown to effectively reduce waste sludge. Also they exhibit a more stable growth on sludge than other worms, and moreover they replicate asexually. The latter aspect makes the processing of a predation reactor more easy.

In the method according to the invention the worms are exposed on at least one side of the carrier to an escape reflex-inducing stimulus. With an escape reflex-inducing stimulus is meant a stimulus, capable of inducing an escape reflex in aquatic worms exhibiting a swimming motion, which are used in the method. The term escape reflex comprises (rapid) contraction of the anterior and/or posterior end of the worm in response to a stimulus.

Such an escape reflex-inducing stimulus may comprise contacting, vibration or a chemical stimulus. Of the chemical stimuli so far it is only described that this occurs only in response to toxins. The escape reflex, which is induced in the method according to the invention is such that the worm biomass at least partially is released from the carrier. Meaning that at least a fraction of the total population of worms is released from the carrier.

The released worm biomass is subsequently carried off for further processing. Further processing comprises amongst others for example purification and/or processing of the worm biomass to a product. The worm biomass may for example be used for the preparation of amongst others fish feed, binder for example for agricultural chemicals, adhesive, surfactants, coatings, biological degradeable plastics, detergents. Also the worms may directly or indirectly be used as a toxicity organism, as a enzyme source, as protein rich additive or as a fertiliser.

The escape reflex-inducing stimulus may comprise a concentration change of a chemical compound. Preferably the concentration change is a concentration increase. The chemical compound may be any chemical compound of which it is known, or for which the skilled person may easily determine that it may induce an escape reflex in aquatic worms, exhibiting a swimming motion, from the subclass of the *Oligochaeta,* preferably from the family of the *Lumbriculidae* or the family of the *Naididae,* such as *Nais variabilis* or *Nais simplex.* More preferably the chemical compound induces an escape reflex in worms form the genus *Lumbriculus,* such as the species *Lumbriculus variegatus* of the genus *Dero*, for example the species *Dero digitata.* Exposure to the concentration change may for example immersing by amercing the carrier with worms in a medium, comprising a differing concentration of the chemical compound relative to the original medium, wherein the worms were present.

Example 1 illustrates an easy method for determining whether a chemical compound may induce an escape reflex in these worms.

The chemical compound preferably is not toxic or mildly toxic for the worms. According to a preferred embodiment, the chemical compound has an LC₅₀ for 96 hours of ≥ 10 mg/l, such as ≥ 40 mg/l, ≥ 100 mg/l, ≥ 500 mg/l, ≥ 1000 mg/l, ≥ 4000 mg/l, preferably ≥ 6000 mg/l. The benefit of using a non-toxic or mildly toxic compound is that there is no detrimental influence to the worms and/or the biological system of the predation reactor. Hereby the worm biomass keeps a high degree of application, for example as a fish feed, and also it is prevented that a polluted waste stream is created. So far it has not been described that non-toxic and moderately toxic compounds may induce an escape reflex in aquatic worms from the subclass of the *Oligochaeta* exhibiting a swimming motion, such as from the family of the *Lumbriculidae* or the family of the *Naididae.* Especially from the genus *Lumbriculus,* such as the species *Lumbriculus variegatus* or the genus *Dero,* for example the species *Dero digitata*.

The chemical compound preferably is a water soluble salt, which more preferably is highly dissolvable in water. A water soluble salt preferably is applied in a aqueous solution. Highly dissolvable salts have a solubility in water of more than 0,7 mol/l at room temperature. Water soluble salts may be selected from salts, comprising as the kation an alkalimetal. Preferably the alkalimetal is selected from sodium or potassium. Also, the kation in a water soluble salt may be formed by an ammonium ion (NH₄)⁺. Water soluble salts may also be formed by selecting as the anion a halide, such as chlorine, fluorine, bromine, iodine. It is also possible to form water soluble salts with an anions with sulfate (SO₄²⁻), sulfite (SO₃²⁻), nitrate (NO₃⁻), nitrite (NO₂⁻), or phosphate (both HPO₄²⁻ and PO₄³⁻). Also a dissociated short-chain fatty acid (C₁-C₆) may form the anion in a water soluble salt. Examples of such short-chain fatty acids are acetate, lactate, propionate, butyrate, citrate, etc... Examples of water soluble salts are NaCl, NH₄Cl, (NH₄)₂SO₄, KCl, K₂SO₄, Na-acetate, Na₂SO₄ and Na₂HPO₄. A selection from the water soluble salt as NaCl in one embodiment of the invention has special preference. This is because NaCl is readily available, exhibits a beneficial escape reflex- inducing stimulus, is non-toxic for worms to a high concentration, and has a low environmental load. It has been described that the LC₅₀ after 96 hours for *Lumbriculus variegatus* for NaCl is 7,7 g/l. U.S. Environmental Protection Agency, preliminary investigation of the extent and effect of sediment contamination in white lake, MI, Appendix E, Acute toxicity reference data with *Lumbriculus variegatus* preliminary toxicity screening and bioaccumulation test of whit lake sediments). For *Dero digitata* the 24 hour LC₅₀ for NaCl is 6.8 g/l (Mischke et al, 2001, "Acute toxicity of several chemicals to the oligochaete Dero digitata", Journal of the world aquaculture society 32 (2); 184-188).

The carrier in a preferred embodiment of the invention is provided in a predation reactor. In such a predation reactor, the waste sludge is contacted with at least one side of the porous carrier. An example of a type of reactor is disclosed in the international patent application PCT/NL2006/050115.

According to a further embodiment of the method, the escape reflex-inducing stimulus comprises a suitable mechanical and/or electrical stimulus. As is known to the skilled person an escape reflex may also be induced in aquatic worms from the subclass of the *Oligochaeta* having a swimming motion, such as from the family of the *Lumbriculidae,* for example from the genus *Lumbriculus,* in particular the species *Lumbriculus variegatus* by means of a mechanical and/or electrical stimulus. A mechanical stimulus may by induced by contacting the worms at at least one side of the carrier. For example by moving a brush along the surface of one side of the carrier.

According to a further preferred embodiment of the method the predation reactor comprises a first and a second compartment, divided by a carrier, preferably a porous carrier. In this predation reactor an oxygen containing aqueous flow (for example effluent) is provided to the first compartment and waste sludge is provided to the second compartment. Hereby the worms will direct themselves with their anterior end (where their mouth is situated) to the second compartment and with their posterior end (where oxygen intake takes place) to the first compartment.

In a further embodiment of the method exposure to the escape reflex-inducing stimulus takes place periodically during 1 second to 30 minutes after a predetermined increase of the worm biomass. This exposure preferably is 10 seconds to 15 minutes, more preferably 30 seconds to 5 minutes, most preferably 45 seconds to 1 minute.

In a preferred embodiment a harvest criterium is determined in advance, which is used as a criterium to commence with the exposure to the escape reflex-inducing stimulus. The harvest criterium may for example be determined in relation to an elapsed time span or an increase of the worm biomass. During and/or after exposure to the escape reflex-inducing stimulus, the worm biomass in the porous carrier will be decreased. After the harvest criterium again has been met, exposure to the escape reflex-inducing stimulus may again occur. By using this embodiment thus the harvesting of the worm biomass by exposure to the escape reflex-inducing stimulus is determined and the amount of worm biomass in the porous carrier is regulated.

The invention further relates to a predation reactor suitable for performing the method according to the invention. The predation reactor comprises a predation compartment whereto waste sludge is provided and where the worms have access to the sludge. The waste sludge is provided to the predation reactor with means known to the skilled person, such as a conduit or a different water way system. Similarly, the predated waste sludge is removed from the predation compartment with such means known to the skilled person.

The predation compartment is in such contact with the carrier, for example a porous carrier, comprising an amount of biomass of oligochaete aquatic worms having a swimming motion, that the (porous) carrier is exposed to waste sludge at least on one side. Hereby the waste sludge flows along or through the carrier wherein the worms are present. This makes it possible for the worms to feed in and/or from within the carrier with waste sludge.

As a carrier a material may be applied which comprises pores, openings or recesses wherein or whereon the aquatic worms having a swimming motion may nest. Examples of suitable carriers are flexible spungelike materials, such as Recticel^{®} or weblike materials. According to a preferred embodiment, a porous carrier is applied which more preferably has an average pore width of 100*µ*m to several mm, such as 100-3000 µm, for example 200-1000 *µ*m, more preferably 200-500 *µ*m.

The aquatic worms having a swimming motion may be selected from the subclass of the *Oligochaeta,* preferably from the family of the *Lumbriculidae* or the family of the *Naididae,* such as *Nais variabilis* or *Nais simplex.* More preferably the worms are selected from the genus *Lumbriculus,* such as the species *Lumbriculus variegatus* or the genus *Dero,* for example the species *Dero digitata.*

The predation reactor further comprises means for providing on at least one side of the carrier an escape reflex-inducing stimulus to the worms. These means in one embodiment of the invention comprise means for providing a concentration change, for example means for providing a concentration increase of a chemical substance. The skilled person will understand how to provide a concentration change of a chemical substance. In particular, the skilled person will understand that addition of a chemical substance will provide a concentration increase of the added substance. Addition of a chemical substance may be by means of adding a concentrated solution of the chemical substance.

In a different embodiment of the predation reactor, the means for providing the escape reflex-inducing stimulus are means for providing a suitable mechanical and/or electrical stimulus. A mechanical stimulus may be induced by contacting the worms on at least one side of the carrier. This may for example be achieved by moving a brush along the surface of one side of the carrier.

Furthermore, the predation reactor comprises means for removing worm biomass, which is released from the porous carrier under the influence of the escape reflex-inducing stimulus. The released worm biomass for example is removed for further processing. Further processing comprises amongst others for example cleaning and/or processing of the worm biomass to a product. The worm biomass may for example be used for preparing amongst others fish feed, binder for example for agricultural chemicals, adhesives, surfactants, coatings, biologically degradeable plastics, detergents. Also the worms may directly or indirectly be used as a toxicity organism, as an enzyme source, as a protein with additive or as a fertiliser. Suitable means for removing the worm biomass will be known to the skilled person. For example, the worm biomass may be removed by pumping together with a fluid. Alternatively, the worm biomass may be removed by means of a conveyor belt.

The above mentioned and other technical features of the predation reactor according to the invention should be understood in view of the above description of the method according to the invention.

The invention will further be explained with reference to the following examples, which are not intended to restrict the invention in any way.

### Example 1

Individual worms of a species to be tested are transferred from a stock container, comprising aerobic waste sludge (the substrate to be degraded), to a petri dish comprising distilled water comprising a dissolved amount of a chemical substance, possibly inducing an escape reflex. If an escape reflex occurs, this is simply perceptible as a rapid movement of the worm by means of twisting and/or swimming. Compounds which have shown in this procedure that they may induce an escape reflex, may further be tested. This may be by transferring individual worms from the stock container to a petri dish with effluent from a purification plant wherefrom the aerobic purification sludge originates comprising a dissolved amount of a chemical substance, for which previously it was shown that it may induce an escape reflex. This to show whether under these circumstances the escape reflex also occurs. When the escape reflex does not occur, the stimulus, that possibly induces the escape reflex, may be increased further, for example by increasing the concentration of the chemical substance.

### Example 2

With the method described in example 1, it was investigated whether NaCl may induce an escape reflex in *Lumbriculus variegatus.* NaCl was tested with the addition of 0,125, 0,25, 0,5 and 1 g/l. All worms transferred to demineralised water before they were exposed to NaCl, showed an escape reflex under all tested concentrations. The worms, transferred from effluent to demineralised water with the same additions of NaCl, only responded to concentrations higher than 0,125 g/l NaCl.

### Example 3

A sponge-like carrier material (Recticel) having a mean pore width of 0.5-2 mm wherein worms of the species *Lumbriculus variegatus, Dero digitata* and *Nais sp.* had nested was submerged in demineralised water containing 1 g/l NaCl. Directly after submersion of the sponge-like carrier, the major part of the worms left the carrier with vigorous swimming motions.

### Example 4

A similar test as described in example 3 was carried out with only the spongelike carrier. However, instead of NaCl 1 g/l KCl was added to the demineralised water. A similar effect was observed as in example 3, however there was some delay in the moment when the worms "fled" from the carrier.

## Claims

1. Method comprising:
(i) providing a carrier, for example a porous carrier, comprising a number of aquatic worms from the order of the *Oligochaeta* exhibiting a swimming motion;
(ii) exposing the worms on at least one side of the carrier to an escape reflex-inducing stimulus, such that the worm biomass at least partially is released from the carrier;
(iii) removing the worm biomass, released from the porous carrier for further processing.

2. Method according to claim 1, wherein the escape reflex-inducing stimulus comprises a concentration change, preferably a concentration increase of the chemical substance.

3. Method according to claim 2, wherein the chemical substance has a LC₅₀ for 96 hours for the worms of ≥10 mg/l, such as ≥40 mg/l, ≥100 mg/l, ≥500 mg/l, ≥1000 mg/l, ≥4000 mg/l, preferably ≥6000 mg/l.

4. Method according to claims 2-3, wherein the chemical substance comprises a water soluble salt, preferably a highly dissolvable salt.

5. Method according to any of the claims 2-4, wherein the concentration change comprises a concentration increase of the chemical substance to 6000 mg/l, preferably to 200 mg/l, more preferably 100 mg/l, most preferably 50 mg/l.

6. Method according to claim 4, wherein the salt is selected as NaCl.

7. Method according to any of the claims 1-6, wherein the carrier is provided in a predation reactor, wherein waste sludge is contacted with at least one side of the carrier.

8. Method according to claim 1, wherein the escape reflex-inducing stimulus comprises a suitable mechanical and/or electrical stimulus.

9. Method according to claims 1-8, wherein the predation reactor comprises a first and a second compartment, separated by the carrier, for example preferably a porous carrier, wherein an oxygen containing aqueous flow is provided to the first compartment and waste sludge is provided to the second compartment and wherein the worms are exposed to the escape reflex-inducing stimulus on the side of the second compartment.

10. Method according to any of the claims 1-9, wherein exposure to the escape reflex-inducing stimulus occurs periodically during 1 second to 30 minutes, preferably 10 seconds to 15 minutes, more preferably 30 seconds to 5 minutes, most preferably 45 seconds to 1 minute after a predetermined increase of the worm biomass.

11. Predation reactor, comprising a predation compartment, means for providing waste sludge to the predation compartment, means for removing predated waste sludge from the predation compartment, which predation compartment is provided with a carrier preferably a porous carrier, comprising an amount of aqueous worms from the class of the *Oligochaeta,* exhibiting a swimming motion, such that at least one side of the porous carrier is exposed to waste sludge, **characterized in that** the predation reactor further comprises means for providing an escape reflex-inducing stimulus to the worms on at least one side of the carrier, and means for removing worm biomass released from the porous carrier under influence of the escape reflex-inducing stimulus.

12. Predation reactor, according to claim 11, wherein the means for providing the escape reflex-inducing stimulus to the worms comprise means for providing a concentration change, preferably a concentration increase of a chemical substance.

13. Predation reactor according to claim 12, wherein the chemical substance has an LC₅₀ for 96 hours for the worms of ≥10 mg/l, such as ≥40 mg/l, ≥100 mg/l, ≥500 mg/l, ≥1000 mg/l, ≥4000 mg/l, preferably ≥6000 mg/l.

14. Predation reactor according to claims 11-13, wherein the chemical substance is a water soluble salt, preferably a higly dissolvable salt.

15. Predation reactor according to claim 12-14, wherein the concentration change comprises a concentration increase of the chemical substance to 6000 mg/l, preferably to 200 mg/l, more preferably 100 mg/l, most preferably 50 mg/l.

16. Predation reactor according to claims 14-15, wherein the salt is selected as NaCl.

17. Predation reactor according to claim 11, wherein the means for providing the escape reflex-inducing stimulus to the worms comprises means for providing a suitable mechanical and/or electrical stimulus.

18. Predation reactor according to claims 11-17, wherein the predation reactor comprises a second compartment, and the second compartment and the predation compartment are separated by the carrier, wherein furthermore means are present to provide an oxygen comprising aqueous flow to the second compartment.

19. Predation reactor according to claims 11-18, wherein the means for exposing the worms to the escape reflex-inducing stimulus on at least one side of the porous carrier are designed to expose the worms to the stimulus for 1 second to 30 minutes, preferably 10 seconds to 15 minutes, more preferably 30 seconds to 5 minutes, most preferably 45 seconds to 1 minute, preferably after a preselected harvesting criterium is fulfilled.
